Europäisches Patentamt

(19) European Patent Office

Office européen des brevets

(11) Veröffentlichungsnummer: **0 146 103**
**B1**

(12) **EUROPÄISCHE PATENTSCHRIFT**

(45) Veröffentlichungstag der Patentschrift:
05.08.87

(51) Int. Cl.⁴: **A 23 L 1/226,** A 23 L 1/235,
C 11 B 9/00, A 61 K 7/46

(21) Anmeldenummer: 84115114.5

(22) Anmeldetag: 10.12.84

(54) Verwendung von 5-Methyl-2-hepten-4-on als Riech- und/oder Aromastoff sowie diesen Stoff enthaltende Riech- und/oder Aromastoffkompositionen.

(30) Priorität: 17.12.83 DE 3345784

(43) Veröffentlichungstag der Anmeldung:
26.06.85 Patentblatt 85/26

(45) Bekanntmachung des Hinweises auf die Patenterteilung:
05.08.87 Patentblatt 87/32

(84) Benannte Vertragsstaaten:
AT CH DE FR GB IT LI NL SE

(56) Entgegenhaltungen:
EP - A - 0 073 301
US - A - 4 315 911
US - A - 4 337 277

J.C. LEFFINGWELL et al.: "Tobacco Flavoring for Smoking Products", 1972, Seiten 40,43, R.J. Reynolds Tobacco Co., Winston-Salem, N.C., US;
S. ARCTANDER: "Perfume and Flavor Chemical (Aroma Chemicals)", II, 1969, S. Arctander, Montclair, N.J., US;
Vortrag von P. Cabella et al. anlässlich der National Conference on Flavourings, Pavia, 8-10. Mai, 1986, Erklärung von P. Cabella

(73) Patentinhaber: Haarmann & Reimer GmbH,
Postfach 1253, D-3450 Holzminden (DE)

(72) Erfinder: Emberger, Roland, Dr., Bärenfang 4,
D-3450 Holzminden (DE)
Erfinder: Köpsel, Manfred, Dr., Schinkelstrasse 1,
D-3450 Holzminden (DE)
Erfinder: Brüning, Jürgen, Dr., Sparenbergstrasse 39,
D-3450 Holzminden (DE)
Erfinder: Hopp, Rudolf, Dr., Auf dem Gehrenkamp 28,
D-4350 Holzminden (DE)
Erfinder: Sand, Theodor, Dr., Vogelsang 3,
D-3450 Holzminden (DE)

(74) Vertreter: Mann, Volker, Dr. et al, c/o Bayer AG
Konzernverwaltung RP Patentabteilung,
D-5090 Leverkusen 1, Bayerwerk (DE)

## Beschreibung

Die Erfindung betrifft die Verwendung von 5-Methyl-2-hepten-4-on als Riech- und/oder Aromastoff, sowie diesen Stoff enthaltende Riech- und/oder Aromastoffkompositionen.

5-Methyl-2-hepten-4-on wird im Rahmen der Untersuchung von Bedingungen, unter denen Ketole durch partielle Hydrierung von 1,3-Diketonen erhalten werden können, als Zwischenprodukt bei der Herstellung des gesättigten Ketons, das zur Bestimmung der Ketolstruktur verwendet wurde, beschrieben [J. Am. Chem. Soc. 61, 3303 (1939)].

In Tetrahedron Letters 23, 335 (1982) wird ein Reaktionsgemisch beschrieben, das aus 4 Teilen 5-Methyl-2-hepten-4-on und 1 Teil 5-Methyl-2-hepten-4-on besteht.

Angaben zu den Eigenschaften von 5-Methyl-2-hepten-4-on sind nicht bekannt.

Es wurde die Verwendung von 5-Methyl-2-hepten-4-on als Riech- und/oder Aromastoff gefunden.

Es wurden ausserdem Riechstoffkompositionen und/oder Aromastoffkompositionen gefunden, die 5-Methyl-2-hepten-4-on enthalten.

Das erfindungsgemäss zu verwendende 5-Methyl-2-hepten-4-on ist ein wertvoller Riechstoff und/oder Aromastoff, der in höherer Konzentration eine nussartige, insbesondere an Haselnuss erinnernde Note aufweist, in grösserer Verdünnung eine Enhancer-Wirkung aufweist und Riechstoffkompositionen eine grössere Natürlichkeit verleiht, wobei es in Mengen von 0,01 bis 10 Gew.-%, bezogen auf die gesamte Komposition eingesetzt werden kann.

Das erfindungsgemäss zu verwendende 5-Methyl-2-hepten-4-on kann beispielsweise durch Umsetzung von 2-Butylmagnesiumbromid (hergestellt z.B. aus 2-Brombutan und Magnesiumspänen) in etherischer Lösung mit Crotonaldehyd erhalten werden. Man erhält hierbei als Zwischenprodukt 5-Methyl-2-hepten-4-ol, das mit einem Gemisch aus Natriumdichromat/Schwefelsäure zu 5-Methyl-2-hepten-4-on oxidiert wird. Dabei fällt das 5-Methyl-2-hepten-4-on als Gemisch der cis-trans-Isomeren an, wobei das trans-Isomere überwiegt. Das Isomerengemisch kann durch einfache Trennmethoden, wie z.B. fraktionierte Destillation über eine Füllkörperkolonne bzw. Spaltrohrkolonne oder präparative Gaschromatographie, in das cis- und das trans-Isomere getrennt werden. Es hat sich aber gezeigt, dass die Verwendung der einzelnen Isomeren keine Vorteile gegenüber der Verwendung des Gemisches bringt.

Der erfindungsgemäss zu verwendende Riechstoff wird in Kombination mit anderen, an sich bekannten Riechstoffen [Arctander, Perfume and Flavor Chemicals, Montclair, N.J. (USA), 1969] und etherischen Ölen [Arctander, Perfume and Flavor Materials of Natural Origin., Elisabeth, N.J. (USA), 1960] angewendet und führt zu Parfümbasen und Riechstoffkompositionen mit ausdrucksstarken Noten, die sich hervorragend für die Parfümierung von Fertigprodukten des Aerosol-, Waschmittel- und des chemisch-technischen Sektors, insbesondere aber des Feinparfümerie- oder Kosmetiksektors eignen, z.B. für Detergentien, Haarpflegemittel, Schaumbäder, Badesalz, Geschirrspülmittel, Waschpulver, Seifen, Antiperspirans, Puder, Cremes, Rasierwasser, After-Shave-Lotions, Raumluftverbesserer, WC-Reiniger, Raum-Sprays, Antiperspirans-Sprays, Deodorant-Sprays, Körper-Sprays, Insektizid-Sprays und Sonnenschutzmittel.

Die Herstellung der Parfümkompositionen und parfümierten Produkte erfolgt in üblicher Weise, beispielsweise durch Zusammengeben der Komponenten.

Weiterhin ist das erfindungsgemäss zu verwendende 5-Methyl-2-hepten-4-on ein wertvoller Aromastoff, der sich durch überraschend niedrige Geschmacksschwellenwerte auszeichnet.

So liegt in jeweils 3%iger Saccharose-Lösung die Wahrnehmungsschwelle bei etwa $5 \times 10^{-6}$ ppm und die Erkennungsschwelle bei etwa $30 \times 10^{-6}$ ppm.

An der Wahrnehmungsschwelle ergibt 5-Methyl-2-hepten-4-on einen Geschmack der als «weich, buttrig, volles Mundgefühl» bezeichnet werden kann, bei der und über der Erkennungsschwelle lautet die Geschmacksbeschreibung «nussig, Haselnuss, weichbuttrig, volles Mundgefühl». Neben seiner spezifischen Charakterisierung in Richtung Haselnuss in entsprechenden Aromakompositionen wirkt es in allen Nicht-Nusstypen insbesondere abrundend und mehr Natürlichkeit vermittelnd durch seine buttrig-weiche Grundfülle.

Die unter Verwendung von 5-Methyl-2-hepten-4-on hergestellten Aromakompositionen können im gesamten Nahrungsmittel- und Genussmittelbereich, Mundpflege und Tierfutter eingesetzt werden. Insbesondere sind sie geeignet für Fondantmassen, Geleefrüchte, Hartkaramellen, Toffeemassen, Schokoladenmassen, Nougatmassen, Fettmassen, Margarine, Speiseöl, Kuchenmehle, Biskuitmassen, Backwaren, Extrusionsprodukte, Milchprodukte, Sauermilchprodukte, Getränke, Speiseeis, Gummi, Mundpflegemittel, Tabakprodukte, Fertiggerichte, Fleisch- und Wurstprodukte, Suppen, Sossen, Gemüsekonserven, Spirituosen, pflanzliche und mikrobielle Proteine und alle Arten von industriell gefertigtem Tierfutter.

Das erfindungsgemässe 5-Methyl-2-hepten-4-on wird in Mengen von $5 \times 10^{-6}$ ppm bis 100 ppm, vorzugsweise $3 \times 10^{-5}$ ppm bis 10 ppm, bezogen auf das verzehrfertige Nahrungsmittel verwendet.

Herstellung:

*Beispiel 1*

Aus 24,32 g Magnesiumspänen und 137 g 2-Brombutan wird in 100 ml Ether 2-Butylmagnesiumbromid hergestellt. Zu dieser Lösung werden bei 0 bis 10°C 56 g Crotonaldehyd, gelöst in 60 ml Ether, zugetropft. Anschliessend wird 2 Stunden unter Rückfluss gekocht. Dann wird abgekühlt, der Ansatz mit Eiswasser/Salzsäure zersetzt, die organische Phase extrahiert, neutralisiert und destilliert. Es werden 56 g Methyl-2-hepten-4-ol mit einem Kp. 70°C/18 mb erhalten. Zu 52 g dieses Alkohols wird unter Eiskühlung langsam ein Gemisch aus 40,34 g Natriumdichromat und 54,1 g Schwefelsäure gegeben. Nach einer Stunde Nachreaktion bei Raumtemperatur wird mit Ether extrahiert, die organische Phase neutralgewaschen und das Lösungsmittel abgezogen und das Rohprodukt destilliert. Durch Redestillation an einer Spaltrohrkolonne werden 17,2 g 5-Methyl-2-hepten-4-on vom Kp. 72°C/20 mb erhalten.

Anwendung:

*Beispiel 2*

Eine Parfümkomposition mit Orangennote wird durch Mischen folgender Komponenten hergestellt:

| | |
|---|---|
| Orangenöl weiss | 650 |
| Linalylacetat | 325 |
| Styrolylacetat | 20 |
| Nootkaton 95% | 5 |

1000 Gewichtsteile

Durch Zugabe von 1 Gewichtsteil 5-Methyl-2-hepten-4-on bekommt die Komposition eine sehr natürliche, mandarinen- und orangenschalige Note.

*Beispiel 3*

Eine Aromakomposition A mit Apfelaroma wird durch Mischen der folgenden Bestandteile hergestellt:

| | |
|---|---|
| Acetaldehyd | 10 |
| n-Butylacetat | 50 |
| trans-2-Hexenal | 30 |
| Ethylacetat | 30 |
| Hexalacetat | 20 |
| n-Buttersäure | 10 |
| Propylenglycol | 850 |

1000 Gewichtsteile

Eine Aromakomposition B wird durch Zugabe von 0,002% 5-Methyl-2-hepten-4-on zu A hergestellt.

A und B werden in einer Dosierung von 7,5 ppm zu einer Lösung von 5% Saccharose und 0,05% Citronensäure in Wasser gegeben und einer Geschmacksprüfung unterzogen. Dabei wird Komposition B der Komposition A eindeutig vorgezogen. Das Aroma der Komposition B wird als voller, natürlicher, saftiger und typischer in Richtung reifer Apfel beschrieben.

*Beispiel 4*

Eine Aromakomposition C mit Vanillearoma wird durch Mischen folgender Bestandteile hergestellt:

| | |
|---|---|
| Vanillin | 50 |
| Ethylvanillin | 10 |
| Heliotropin | 2 |
| Diacetyl | 2 |
| Propylenglycol | 936 |

1000 Gewichtsteile

Eine Aromakomposition D wird durch Zugabe von 0,004% 5-Methyl-2-hepten-4-on zu C hergestellt.

C und D werden in einer Dosierung von 37,5 ppm zu einer Lösung von 5% Saccharose in Wasser gegeben und einer Geschmacksprüfung unterzogen. Dabei wird Komposition D der Komposition C deutlich vorgezogen. Das Aroma der Komposition D wird als voller, weicher und natürlicher beschrieben.

*Beispiel 5*

Eine Aromakomposition E mit Walnussaroma wird durch Mischen folgender Bestandteile hergestellt:

| | |
|---|---|
| Vanillin | 10 |
| Methylcyclopentenolon | 30 |
| Resorcindimethylether | 30 |
| Maltol | 5 |
| Propylenglycol | 945 |

1000 Gewichtsteile

Eine Aromakomposition F wird durch Zugabe von 0,003% 5-Methyl-2-hepten-4-on zu E hergestellt.

F und E werden in einer Dosierung von 75 ppm zu einer Lösung von 5% Saccharose in Wasser gegeben und einer Geschmacksprüfung unterzogen. Dabei wird Komposition F der Komposition E deutlich vorgezogen. Das Aroma der Komposition F wird als voller, weicher und typischer in Richtung Walnuss beschrieben,

*Beispiel 6*

Eine Aromakomposition G mit Haselnussaroma wird durch Mischen folgender Bestandteile hergestellt:

| | |
|---|---|
| Vanillin | 30 |
| Benzaldehyd | 10 |
| Furfurol | 5 |
| 2-Ethyl-3,5(3,6)-dimethylpyrazin | 5 |
| 2-Methyl-3-ethyl-pyrazin | 5 |
| Resorcindimethylether | 50 |
| Propylenglycol | 895 |

1000 Gewichtsteile

Eine Aromakomposition H wird durch Zugabe von 0,05% 5-Methyl-2-hepten-4-on zu G hergestellt.

G und H werden in einer Dosierung von 15 ppm zu einer Lösung von 5% Saccharose in Wasser gegeben und einer Geschmacksprüfung unterzogen. Dabei wird Komposition H der Komposition G deutlich vorgezogen. Das Aroma der Komposition H wird als voller, natürlicher und wesentlich typischer in Richtung Haselnuss beschrieben.

*Beispiel 7*

Eine Aromakomposition I mit Pilzaroma wird durch Mischen folgender Bestandteile hergestellt:

| | |
|---|---|
| Octen-1-ol-3 | 40 |
| Hexanol | 5 |
| Propylenglycol | 955 |

1000 Gewichtsteile

Eine Aromakomposition J wird durch Zugabe von 0,01% 5-Methyl-2-hepten-4-on zu I hergestellt.

I und J werden in einer Dosierung von 7,5 ppm zu einer Lösung von 0,5% Kochsalz in Wasser gegeben und einer Geschmacksprüfung unterzogen. Dabei wird Komposition J der Komposition I deutlich vorgezogen. Das Aroma der Komposition J wird als voller, wesentlich natürlicher und typischer im Pilzcharakter bezeichnet.

**Patentansprüche**

1. Verwendung von 5-Methyl-2-hepten-4-on als Riech- und/oder Aromastoff.

2. Riechstoffkomposition, enthaltend 5-Methyl-2-hepten-4-on.

3. Riechstoffkomposition nach Anspruch 2, gekennzeichnet durch einen Gehalt von 0,01 bis 10 Gew.-% an 5-Methyl-2-hepten-4-on, bezogen auf die gesamte Komposition.

4. Aromastoffkomposition, enthaltend 5-Methyl-2-hepten-4-on.

5. Aromastoffkomposition nach Anspruch 4, gekennzeichnet durch einen Gehalt von $5 \times 10^{-6}$ ppm bis 100 ppm an 5-Methyl-2-hepten-4-on, bezogen auf das verzehrfertige Nahrungsmittel.

**Claims**

1. Use of 5-methyl-2-hepten-one as a perfuming substance and/or flavouring substance.

2. Perfuming composition containing 5-methyl-2-hepten-one.

3. Perfuming composition according to Claim 2, characterised in that it contains 0.01 to 10% by weight of 5-methyl-2-hepten-one, based on the total composition.

4. Flavouring composition containing 5-methyl-2-hepten-one.

5. Flavouring composition according to claim 4, characterised in that is contains $5 \times 10^{-6}$ ppm to 100 ppm of 5-methyl-2-hepten-one, based on the ready-to-consume foodstuff.

**Revendications**

1. Utilisation de la 5-méthyl-2-heptène-4-one comme parfum et/ou aromate.

2. Composition de parfum contenant de la 5-méthyl-2-heptène-4-one.

3. Composition de parfum suivant la revendication 2, caractérisée par une teneur en 5-méthyl-2-heptène-4-one de 0,01 à 10% en poids, par rapport à la composition totale.

4. Composition d'aromate contenant de la 5-méthyl-2-heptène-4-one.

5. Composition d'aromate suivant la revendication 4, caractérisée par une teneur en 5-méthyl-2-heptène-4-one de $5 \times 10^{-6}$ ppm a 100 ppm, par rapport à l'aliment prêt à la consommation.